# EUROPEAN PATENT SPECIFICATION

(11) **EP 3 914 190 B1**
(45) Date of publication and mention of the grant of the patent: **06.09.2023**
(21) Application number: 20702402.7
(22) Date of filing: 22.01.2020
(51) Int. Cl.: A61F 2/16

(54) **INTRAOCULAR LENS INJECTOR WITH SPEED REGULATION**
INTRAOKULARER LINSENINJEKTOR MIT GESCHWINDIGKEITSREGULIERUNG
INJECTEUR DE LENTILLE INTRAOCULAIRE À RÉGULATION DE VITESSE

(30) Priority: 22.01.2019 US 201962795531 P; 31.01.2019 US 201962799654 P
(43) Date of publication of application: 01.12.2021
(73) Proprietor: Carl Zeiss Meditec AG, 07745 Jena (DE)
(72) Inventor: ZACHARIAS, Jaime, 7620089 Santiago de Chile (CL)
(74) Representative: Carl Zeiss AG - Patentabteilung
(86) International application number: PCT/EP2020/000023
(87) International publication number: WO 2020/151908

(56) References cited:
- EP-A1- 2 873 391
- US-A1- 2014 257 317
- US-A1- 2017 172 727
- US-A1- 2017 367 818

## Description

### TECHNICAL FIELD

The disclosure relates to an intraocular lens injector for inserting an intraocular lens (IOL) into a capsular bag of a human eye, and in particular to an intraocular lens injector with a speed regulator system that is based on a mechanical timer mechanism. The disclosure further relates to a method for controllably releasing an IOL with an intraocular lens injector.

### BACKGROUND

IOLs are medical devices implanted inside the eye. With recent advances in IOL technology, cataract surgery has transitioned from being solely a treatment for visual rehabilitation to also being a refractive procedure with the aim of improving visual function and ultimately the patient's quality of life. IOLs that increase visual function are for example aspheric IOLs and toric IOLs. Aspheric IOLs compensate a spherical aberration of the cornea. Toric IOLs correct corneal astigmatism. IOLs also correct distance vision. The performance of new IOL designs highly depends on the position of the IOL in the optical system of the eye. Depending on the type of correction, even little decentration and tilt of an IOL in the eye may decrease visual quality.

Foldable IOLs made from silicone, soft acrylics, and hydrogels have become increasingly popular because of the ability to fold or roll such soft lenses and to insert them through a small incision. Such IOLs typically consist of a small plastic lens with plastic side struts, called haptics, to hold the lens in place within the capsular bag inside the eye. Such an IOL may be, e.g., a single piece (or one-piece) IOL. The haptic material of a single piece IOL is the same as the optical material. There are also three-piece IOLs with haptics made of a material, e.g., polymethylmethacrylate (PMMA) filaments, which is harder than the optical material.

During surgery, the lens of the patient's eye is removed from the capsular bag of the eye and replaced by the IOL. A most common technique for inserting a foldable IOL into the capsular bag of the eye is through an injector. Such injectors use a plunger to "squeeze" the IOL through a cartridge into the eye. Single piece acrylic and silicone plate haptic IOLs are the simplest to use with injectors. These designs have haptics that are sturdy and to a certain extent are resistant to damage from the plunger, as it forcefully pushes the IOL through the cartridge. In addition, common injectors are not reliable and easy to handle.

Two completely different injector systems are known for inserting a soft and foldable lens. One injector system consists of an injector body (a handpiece) and a separate cartridge, whereby the injector body is reusable or disposable, and the separate cartridge is disposable. Another injector system consists of a preloaded injector, which means the lens is stored within the preloaded injector. Typically, the surgeon pushes a plunger with one finger to inject the IOL into the eye while using the other fingers to stabilize and orient the nozzle into a surgical incision. This simultaneous holding and injecting maneuver can reduce control of the speed of the injection, with a probability of a too rapid and uncontrolled insertion leading to complications such as iris rupture, capsule rupture, and vitreous loss just to mention some.

Examples of such prior art injectors are disclosed by US2017/172727, US2017/367818, US2014/257317 andEP2873391.

### SUMMARY

It is an objective of the present disclosure to provide an IOL injector for controllably releasing an IOL with an IOL injector, and in particular an IOL injector that can be handled in an easy, safe, and controllable manner.

It is also an objective of the present disclosure to limit the speed of releasing the IOL to a predetermined maximum speed to avoid fast and uncontrolled IOL injections, to increase accuracy of IOL delivery, and prevent complications.

An advantageous and controlled method to release an IOL from the IOL injector into the eye is described.

The objective is achieved by providing an IOL injector for releasing an IOL to be injected into a capsular bag of an eye, the IOL injector having a proximal end and a distal end, the IOL injector including an injector body defining a longitudinal axis and including a hollow shaft, a spring arranged in the hollow shaft, a cartridge arranged on the injector body and defining a cavity for holding the IOL, an injection nozzle arranged on the cartridge at the distal end of the IOL injector, a channel extending through the injector body, the cavity, the cartridge, and the injection nozzle in an axial direction along the longitudinal axis, a mechanical speed regulator system configured to control a speed of the releasing of the IOL, and a pusher rod engaged via the hollow shaft with the mechanical speed regulator system and arranged in the channel movably in the axial direction by a force generated by the spring to advance the IOL in the axial direction towards the distal end of the IOL injector out of the cavity through the cartridge and the injection nozzle.

The IOL injector further includes a control lever arranged on the injector body in communication with the mechanical speed regulator system, and the mechanical speed regulator system is responsive to an actuation of the control lever. The mechanical speed regulator system includes a first compound gear, a second compound gear, and a third compound gear, the first compound gear includes a first pinion and the hollow shaft includes a rack, the first pinion and the rack form a rack and pinion mechanism to mechanically couple the mechanical speed regulator system with the hollow shaft. The first, second, and third compound gears form a gear train, and the gear train has a speed multiplying configuration.

The mechanical speed regulator system further includes an anchor mechanism with an anchor, and the third compound gear includes an escapement wheel mechanically engaging with the anchor. According to an aspect of the disclosure the anchor mechanism includes an anchor shaft defining a center of oscillation of the anchor, the anchor has an anchor body, and the anchor body is mounted on the anchor shaft and swivels about the anchor shaft.

According to another aspect of the disclosure, the anchor mechanism further includes a pendulum arm arranged on the anchor body and a mass arranged on an end of the pendulum arm facing away from the anchor body.

The invention is set out in the appended set of claims.

### BRIEF DESCRIPTION OF THE DRAWINGS

The disclosure will now be described with reference to the drawings wherein:
FIG. 1 is a perspective view of an IOL injector according to a first exemplary embodiment of the disclosure;
FIG. 2 is a side view of the IOL injector shown in FIG. 1;
FIG. 3 is a top view of the IOL injector shown in FIG. 1;
FIG. 4 is a bottom view of the IOL injector shown in FIG. 1;
FIG. 5 is a cross-sectional side view of the IOL injector shown in FIG. 1;
FIG. 6 is a cross sectional side view of the side of the IOL injector opposite to the side shown in FIG. 5;
FIG. 7 is a side view of the IOL injector shown in FIG. 1 with the housing removed;
FIG. 8 is a perspective side view of the IOL injector shown in FIG. 1 with the housing removed;
FIG. 9 is a perspective top view of an IOL injector according to a second exemplary embodiment of the disclosure;
FIG. 10 is a perspective view of the IOL injector shown in FIG. 9 with a partially removed housing;
FIG. 11 is a perspective view of the IOL injector shown in FIG. 9 with the housing removed;
FIG. 12 is a perspective view of the IOL injector shown in FIG. 9 with a safety tab in a lock position;
FIG. 13 is a perspective view of the IOL injector shown in FIG. 9 with a safety tab in an operate position;
FIG. 14 is a side view of the IOL injector shown in FIG.9;
FIG. 15 is a side view of an IOL injector according to a third exemplary embodiment of the disclosure;
FIG. 16 is a side view of the IOL injector shown in FIG. 15 with a partially removed housing;
FIG. 17 is a side view of the IOL injector shown in FIG.15 with the housing removed;
FIG. 18 is a detailed side view of the timer mechanism of the IOL injector shown in FIG.15;
FIG. 19 is a bottom view of the timer mechanism of the IOL injector shown in FIG. 15;
FIG. 20 is a cross-sectional side view of the timer mechanism of the IOL injector shown in FIG.19 illustrating the braking mechanism;
FIG. 21 is a side view of an IOL injector according to a fourth exemplary embodiment of the disclosure;
FIG. 22 is a detailed side view of the timer mechanism of the IOL injector shown in FIG.21;
FIG. 23 is another detailed side view of the timer mechanism of the IOL injector shown in FIG.21;
FIG. 24 is a side view of an IOL injector showing a spring compression mechanism with a pusher rod;
FIG. 25 is a cross-sectional bottom view of the IOL injector shown in FIG. 24 with the pusher rod unloaded;
FIG. 26 is another cross-sectional bottom view of the IOL injector shown in FIG. 24 with the pusher rod loaded;
FIG. 27 is a side view of the IOL injector shown in FIG. 26 with a partially removed housing showing a spiral spring; and
FIG. 28 is a side view of the IOL injector shown in FIG. 27 with the housing removed showing a spiral spring.

### DESCRIPTION OF EXEMPLARY EMBODIMENTS

Various objects, features, aspects and advantages of the present disclosure will become more apparent from the following detailed description of the exemplary embodiments of the disclosure, along with the accompanying drawings in which like numerals represent like components.

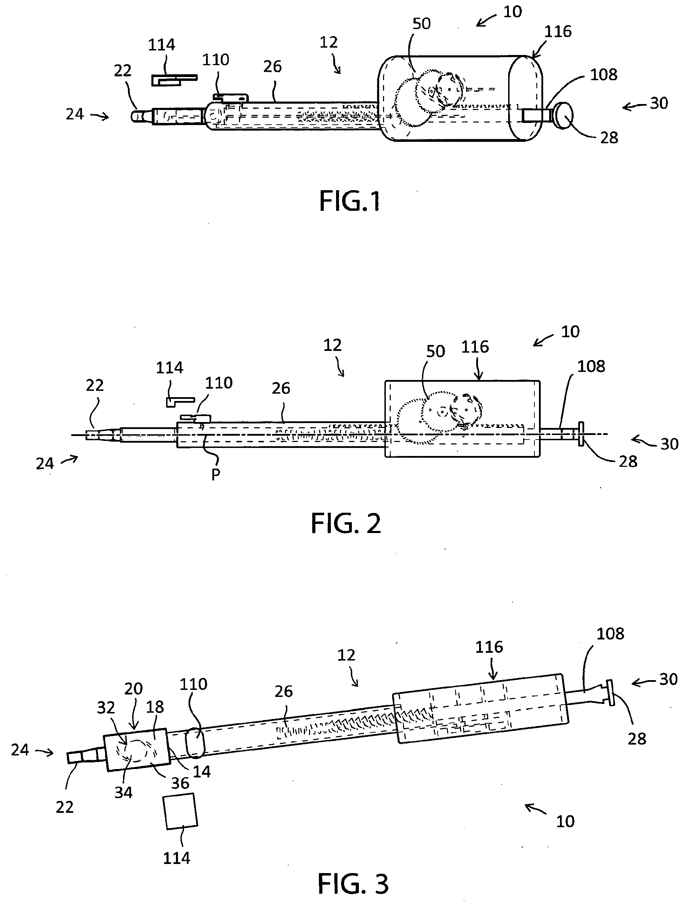

FIGS. 1 to 8 show different views of an IOL injector 10 according to a first exemplary embodiment of the disclosure. The IOL injector 10 has a proximal end 30 and a distal end 24. Injector 10 includes an injector body 12 having a channel 14 extending in a direction of a longitudinal axis P, and a pusher rod 16 slidably inserted in the injector body 12. The injector 10 further includes a cavity 18 for inserting an IOL 32, and a cartridge 20 with an injection nozzle 22 at distal end 24. The injector body 12 has finger grips 26.

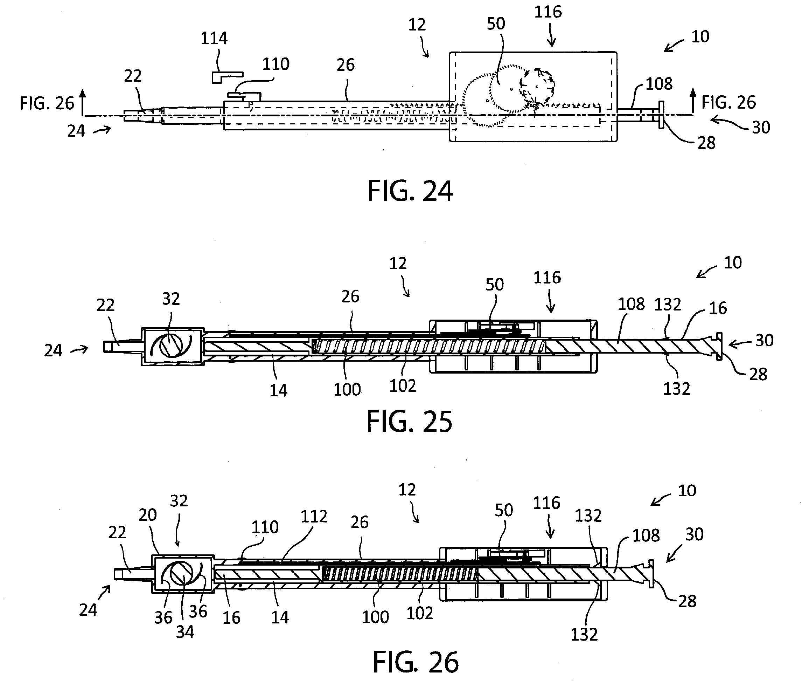

An end of pusher rod 16 opposite from the distal end 24 of the IOL injector 10 mechanically engages with a hollow shaft 102 receiving an axially disposed coiled compression spring 100. Hollow shaft 102 also includes a rack portion 54 of a rack and pinion mechanism 51. Spring 100 can be compressed inside shaft 102 by a spring pusher rod 108 to apply a force onto shaft 102 directed toward the distal end 24. The spring pusher rod 108 can be a fixed part of injector body 12 with spring 100 factory pre-tensed ready for operation. Alternatively, as shown in FIGS. 24 to 26, spring pusher rod 108 can also be an axially slidable part inside the hollow portion of hollow shaft 102 to compress spring 100 when pushed toward the distal end 24 of the IOL injector 10 along longitudinal axis P. A thumb plate 28 on the proximal portion of the spring pusher rod 108 can allow a user to push spring pusher rod 108 to compress spring 100 far enough for operation.

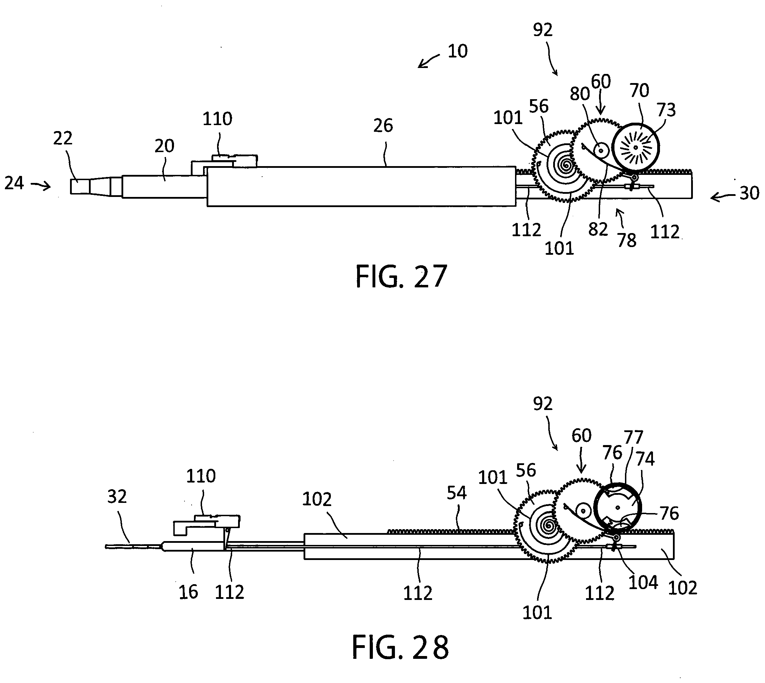

IOL injector 10 further includes pusher rod locking tabs 132 and speed regulator housing 116. The pusher rod locking tabs 132 retain spring pusher rod 108 in a locked position thereby preventing recoil of spring pusher rod 108 after releasing pressure from thumb plate 28 and permitting compressed spring 100 to expand inside the speed regulator housing 116. As an alternative to coil spring 100 directly pushing pusher rod 16, coil spring 100 can be replaced by a pre-tensed spiral spring 101 arranged on one compound gear of the gear train 92, e.g., as shown in FIGS. 27 and 28 to provide mechanical power through pinion 55 and rack 54 to axially displace pusher rod 16 and pushing the IOL toward the eye.

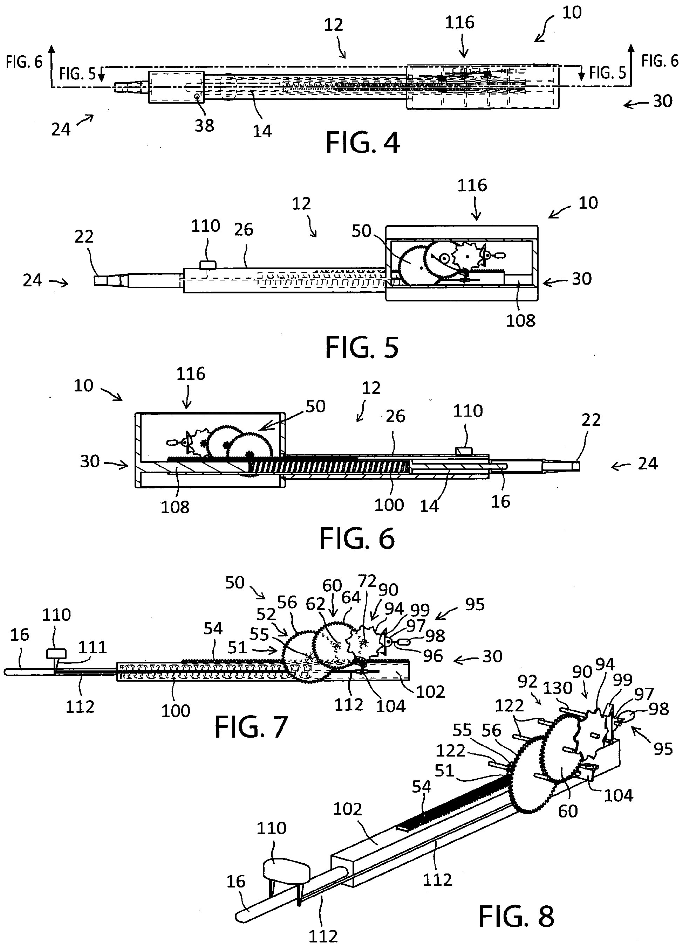

Returning now to FIG. 4. FIG. 4 shows channel 14 extending through the injector body 12, the cavity 18, and the cartridge 20, and which exits at the distal end 24 from injection nozzle 22, such that the pusher rod 16 may advance an IOL 32 positioned in the cavity 18. The term "pusher rod" describes any component advanced through the channel 14 to push an IOL 32 through the cavity 18 and the cartridge 20, such that the intraocular lens 32 exits the IOL injector 10 at the distal end 24 of the injection nozzle 22. In an exemplary embodiment of the disclosure, the injector body 12 may be a reusable hand piece, whereas the cartridge 20, which is releasably connected with the injector body 12, may be a single use part. In another exemplary embodiment, injector body 12, cartridge 20, and cavity 18 may be formed as a single piece from a suitable material, which may include, for example polypropylene or polyethylene. IOL Injector 10 can be made as a fully disposable single use part. Cartridge 20 may also be treated with a lubricious coating, a viscoelastic material and/or liquid in order to facilitate advancement of the IOL within the channel of cartridge 20. Lubricant can be injected in cartridge 20 through a lubricant port 38.

IOL 32 may be any intraocular lens formed of a flexible material, including but not limited to hydrogels, silicone, or acrylic materials. IOL 32 includes as least one optic part 34 and haptics 36 that stably fixate IOL 32 within the eye when implanted. Each haptic 36 has one end anchored in the lens optic part 34 and a free end for attachment to the eye tissue. The optic part 34 is structured to focus light onto a patient's retina and may include any suitable refractive and/or diffractive elements.

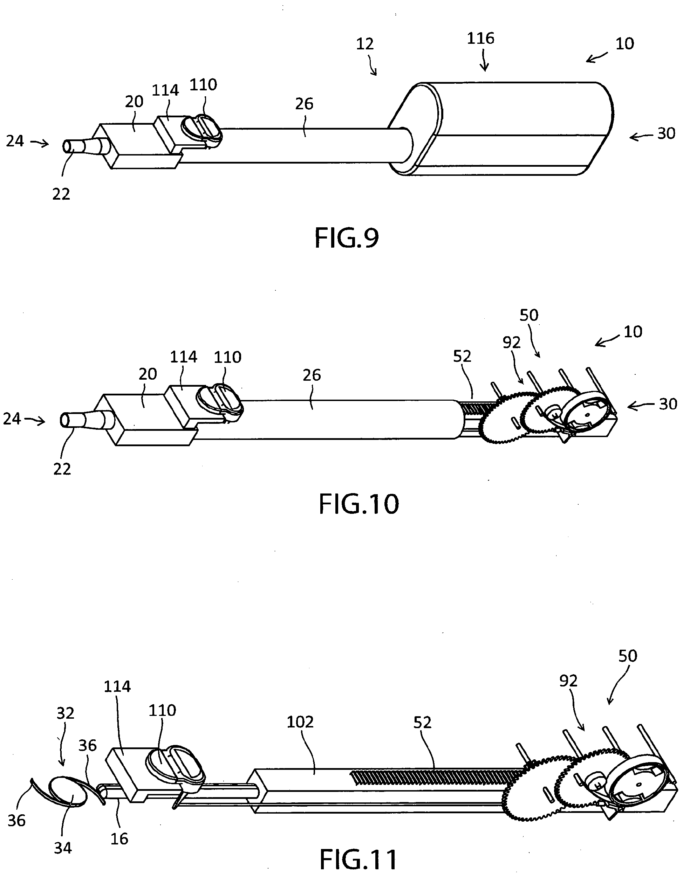

FIGS. 3 and 11 show an IOL 32 positioned in cavity 18 and ready to be inserted from cartridge 20 through nozzle 22 pushed by pusher rod 16 into an eye.

As shown in FIGS. 4 to 8, a mechanical speed regulator mechanism 50 including a reduction gearbox is arranged in a speed regulator housing 116 and mechanically coupled with a rack and pinion mechanism 51 to rack 54 of shaft 102 via a first pinion 55 of a first compound gear 52 having a coaxial first large gear 56. Large gear 56 of compound gear 52 mechanically couples with a second pinion 62 of second compound gear 60 also having a second large gear 64. Second large gear 64 mechanically couples with a third pinion 72 of the third compound gear with an escapement wheel 90. The first, second, and third compound gears 52, 60, and 90 form a gear train 92 in a speed multiplying configuration. The escapement wheel 94 of the compound gear with escapement wheel 90 mechanically engages with an anchor 99 emerging from an anchor body 97. First, second, and third compound gears 52, 60, and 90 are each mounted on respective shafts 122 for expedite rotation. Anchor body 97 is mounted on an anchor shaft 130 defining a center of oscillation of anchor 99. Anchor body 97 also receives a proximal end of a pendulum arm 96. Pendulum arm 96 is attached through the distal end to a mass 98 configuring a pendulum 95. Pendulum arm 96 can be configured to be flexible and can be made, e.g., of a steel filament, a plastic sheet, or other suitable flexible material, in a way that the time-constant of the anchor mechanism can be adjusted by selecting a mass 98 weight, a pendulum arm 96 length, and a pendulum arm 96 spring constant. A finger depressible control lever 110 can be arranged on body 12 for a user to be depressed to activate injector 10 for injecting IOL 32 into a capsular bag of an eye. Lever 110 when depressed perpendicular to axis P can push rearward a rod 112 at the distal end by the action of a wedge 111. Control rod 112 can be slidably disposed in a channel parallel to axis P. Control rod 112 runs through a hole 106 in a pivoting gear lock 104 with a gear lock extension 105 pivotable around a gear lock shaft 124.

Control rod 112 has rod stoppers 120 and 121 at each side of hole 106 in a way that axial displacement of rod 112 initiates a pivoting motion on gear lock 104 around shaft 124. Rod 112 is actuated upon by a prestressed compression spring 113 disposed to push rod stopper 121 in the forward direction towards the distal end 24 of the IOL injector 10. With control lever 110 undepressed, gear lock extension 105 remains positioned against the teeth of large gear 64 impeding rotation of gear 64. With control lever 110 in a depressed state, gear lock extension 105 rotates away from the teeth of large gear 64 allowing rotation of gear 64. A safety tab 114 can be inserted between control lever 110 and body 102 in a way such that unwanted depression of lever 110 is prevented.

To operate the IOL injector 10 shown in FIGS. 1 to 8, an operator may hold injector body 12 with one hand to first inject a volume of through lubricant port 38 that is sufficient to fill channel 14 in the region of IOL 32, cavity 18, and nozzle 22 with the viscoelastic substance. Safety tab 114 can be removed with the other hand by the operator to unlock control lever 110 for operation.

Under visual supervision by the operator, lever 110 can be depressed by one finger of the operator with nozzle 22 at the distal end 24 inserted in a surgical incision for delivering the IOL 32 into an eye, typically filled with viscoelastic substance. Depression of lever 110 produces a rearward displacement of control rod 112 running through hole 106 of pivoting gear lock 104 which has extension 105 engaged with a tooth from gear 64 part of second compound gear 60 preventing gear 60 rotation at rest. The rearward displacement of rod 112 pushes the front rod stopper 120 toward the body of gear lock 104 producing a pivoting motion around shaft 124 that has the effect of displacing gear lock extension 105 away from the teeth of gear 64 unlocking compound gear 60 and allowing it to rotate. The rearward displacement of rod 112 also pushes rear rod stopper 121 further compressing prestressed rod spring 113. Release of lever 110 allows spring 113 to force sliding of rod 112 back into the resting position with extension 105 now pivoting towards gear the teeth of gear 64 and back into a locking position of gear 60. In this way an operator can activate and deactivate IOL injection in a controllable manner.

The speed multiplying gear train 92 composed of first, second, and third compound gears 52, 60, and 90 is powered for rotation by the action of compressed spring 100 inside shaft 102 transmitting force to gear train 92 through rack 54 and pinion 55 mechanism 51. When compound gear 60 is in an unlocked state, rotary force is transmitted to compound gear 90. Escapement wheel 94 of gear 90 forces anchor 99 to oscillate. Anchor 99 limits the speed of rotation of gear 90 according to the period of oscillation of anchor 99. The period of oscillation of anchor 99 can be varied by the length of arm 96, the spring constant of arm 96, and the weight of mass 98. Other factors can also vary the period of oscillation of anchor 99. In a typical configuration, the period of oscillation of anchor 99 can be adjusted to 0.1 seconds (s) (which is the same as a frequency of 10 oscillations per second). The gear train can be configured to advance the pusher rod 16 at a speed of 3 millimeters per second (mm/s) with these settings.

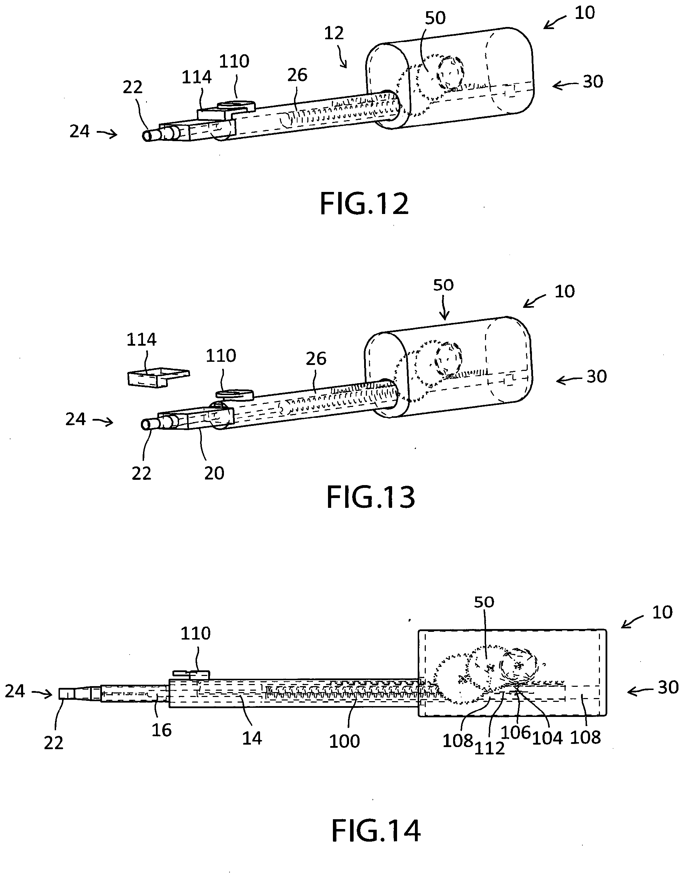

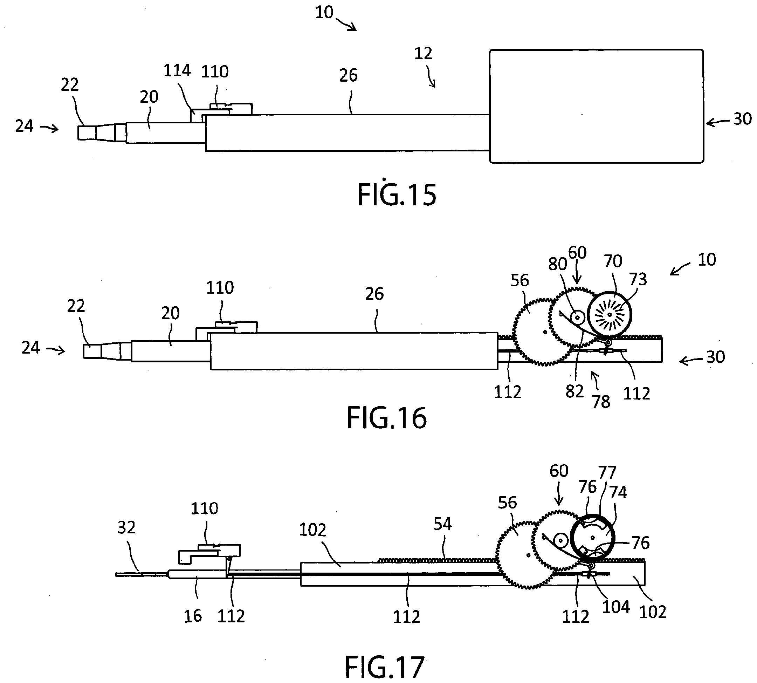

FIGS. 9 to 14 show different views of an IOL injector 10 not forming part of the invention, and FIGS. 15 to 20 show different views of an IOL injector 10 according to another example not forming part of the invention In the exemplary embodiments shown in FIGS. 9 to 20, the third compound gear of escapement wheel 90 is replaced by a third compound gear 70 including flywheel 74. More specifically, compound gear 70 includes third pinion 72 and flywheel 74.

Flywheel 74 introduces an inertial component that allows for incremental axial speed of pusher rod 16 once control lever 110 is depressed by an operator. A maximum speed of rotation of flywheel 74 can be achieved that is within a maximum tolerated speed using the simple flywheel 74 embodiment. The operator can release lever 110 to stop the advancement of pusher rod 16 if the speed is determined to be too fast and the action can be repeated to start again with pusher rod 16 moving from the detent state at incremental speed.

Further control can be obtained by incorporating a speed limiting feature into the flywheel 74. One example of improved control of the maximum speed of rotation of the flywheel 74 by incorporating a speed limiting feature 75 is shown in FIG. 16 where flywheel 74 incorporates air resistive fins 73 which limit the maximum speed of rotation of flywheel 74 based on the principle of increasing air friction with speed of rotation which results in an equilibrium speed of flywheel 74 that can be tailored to the desired maximum speed.

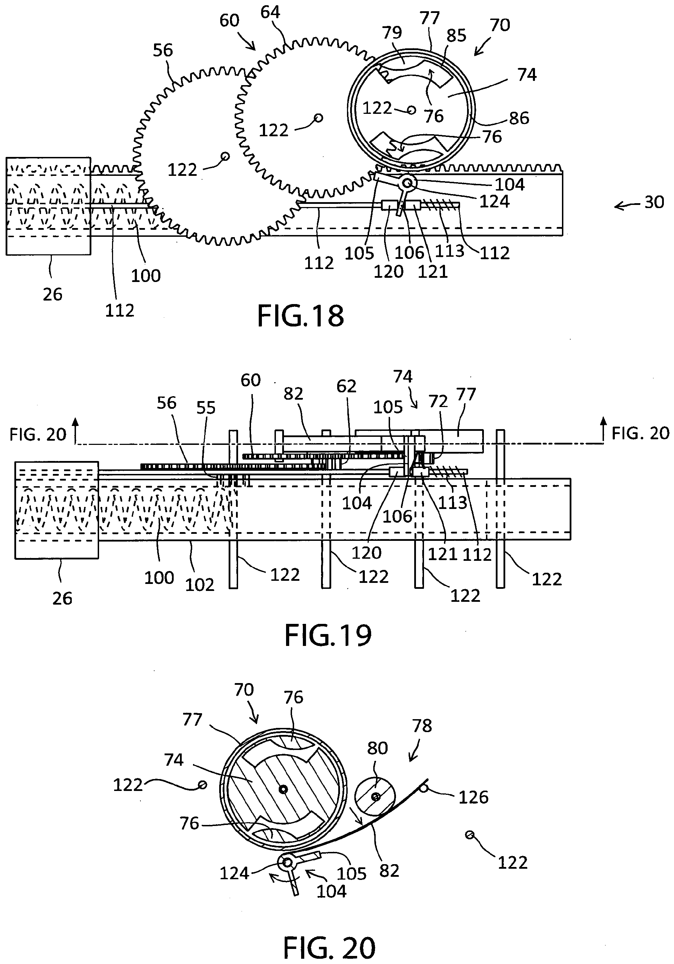

An exemplary embodiment of an improved maximum speed control of flywheel 74 is shown in FIG.18. In this exemplary embodiment, flywheel 74 includes at least one centrifugal speed limiter 76 providing a speed limiting feature. Each of the at least one centrifugal speed limiters 76 includes a flexible arm 85 mounted on the body of flywheel 74 and a centrifugal mass 79 arranged on an end of the radially flexible arm 85 facing away from the body of the flywheel 74. A flywheel shell 77 encircles flywheel 74 defining a circumferential clearance space 86. Flywheel shell 77 can be an extension of speed regulator housing 116. Flywheel 74 can rotate up to a speed where the centrifugal force displaces centrifugal masses 79 thereby bending the radially flexible arms 85 across the clearance space 86. At this critical speed, the centrifugal masses 79 become in contact with flywheel shell 77 and a frictional force slows down flywheel 74 limiting the maximum speed. A maximum flywheel 74 speed can be preset by adjusting the centrifugal masses 79, the flexibility of arms 85, and the dimensions of clearance space 86.

Another exemplary embodiment of an improved speed control is shown in FIGS. 16, 17, and 20. In this exemplary embodiment, depressing lever 110 first releases gear lock extension 105 from the teeth of gear 64. A friction brake 78 is composed of a friction band 82 in contact with a friction wheel 80 built into compound gear 60. Friction band is fixed on one end through a friction band anchor stud 126. The opposite end of friction band 82 is anchored to gear lock 104 in a way such that incremental rotation of lock 104 produces progressive loosening of friction band 82 against friction wheel 80 with a progressive release of brake 78 and a progressive speed of flywheel 74.

In this way, incrementally depressing lever 110 by an operator can produce proportional loosening of band 82 with proportional speed control of flywheel 74 and of pusher rod 16.

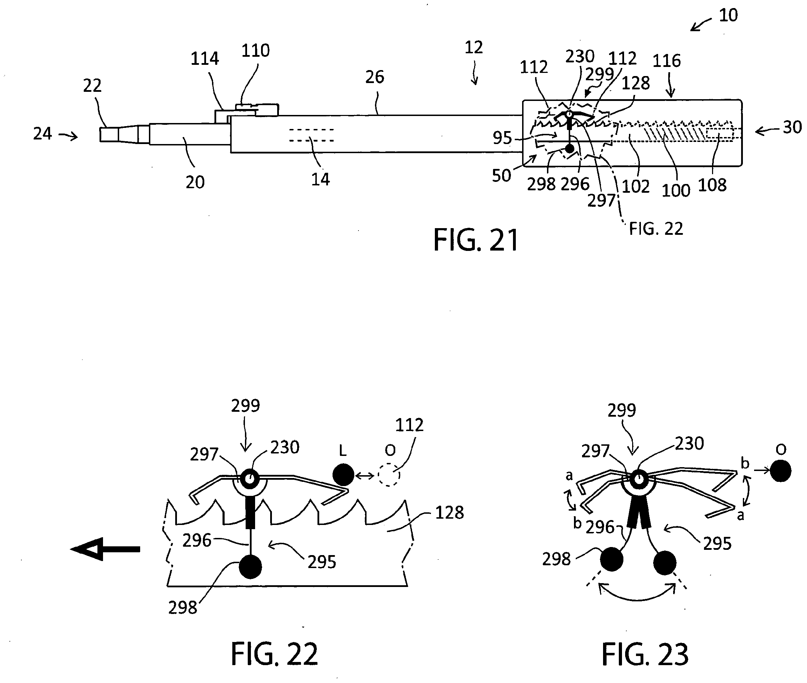

In FIGS. 21 to 23 an IOL injector 10 having a body 12 including a speed regulator mechanism 50 according to another example not forming part of the invention. In this exemplary embodiment, escapement wheel 94 has been replaced by an escapement rack 128 arranged alongside hollow shaft 102. Gear train 92 has been removed and anchor body 297 is mounted on an anchor shaft 230 such that anchor 299 is operatively coupled with escapement rack 128 of hollow shaft 102. By depressing the control lever 110 by an operator, control rod 112 slides rearward from a locked position L blocking oscillation of anchor 299 to an operate position O at which anchor body 297 is released to freely oscillate around the anchor shaft 230 from position "a" to "b" and reverse, see FIG. 23, powered by the force of spring 100 compressed by spring pusher rod 108 and transmitted through hollow shaft 102 to escapement rack 128. Pendulum 295 has a pendulum arm 296 configured to act as a spring, and a pendulum mass 298. The arm length of the pendulum arm 296, the arm spring constant, and the mass value of the mass 298 are adjusted such that the pendulum 295 swings at a rate at which the shaft 110 and the pusher rod 16 advance at a maximum speed, typically at a speed of millimeters per second 3 mm/s.

The injector body 12 holds the cartridge 20. The cartridge 20 can be releasably attached to the injector body 12. The cartridge 20 can be delivered separately. The injector body 12 including pusher rod 16 can be a multiuse device, whereas the cartridge 20 can be a single use device. The injector body 12 and the cartridge 20 can also be manufactured as one single part.

It is understood that the foregoing description is that of the exemplary embodiments of the disclosure and that various changes and modifications may be made thereto without departing from the scope of the disclosure as defined in the appended claims.

### LIST OF REFERENCE NUMERALS

- P: longitudinal axis
- 10: IOL injector
- 12: body
- 14: channel
- 16: pusher rod
- 18: cavity
- 20: cartridge
- 22: nozzle
- 24: distal end
- 26: graspable portion
- 28: thumb plate
- 30: proximal end
- 32: intraocular lens
- 34: optic part
- 36: haptics
- 38: lubricant port
- 50: speed regulator mechanism
- 51: rack and pinion mechanism
- 52: first compound gear
- 54: rack
- 55: fist pinion
- 56: first large gear
- 60: second compound gear
- 62: second pinion
- 64: second large gear
- 70: third compound gear with flywheel
- 72: third pinion
- 73: air resistive fins
- 74: flywheel
- 75: speed limiting feature
- 76: centrifugal speed limiters
- 77: flywheel shell
- 78: friction brake
- 79: centrifugal mass
- 80: friction wheel
- 82: friction band
- 85: radially flexible arm
- 86: clearance
- 90: third compound gear with escapement wheel
- 92: gear train
- 94: escapement wheel
- 95: pendulum
- 96: pendulum arm
- 97: anchor body
- 98: mass
- 99: anchor
- 100: spring
- 101: spiral spring
- 102: hollow shaft
- 104: pivoting gear lock
- 105: gear lock extension
- 106: hole
- 108: spring pusher rod
- 110: control lever
- 111: wedge
- 112: control rod
- 113: rod spring
- 114: safety tab
- 116: speed regulator housing
- 120: front rod stopper
- 121: rear rod stopper
- 122: gear shaft
- 124: gear lock shaft
- 126: friction band anchor stud
- 128: escapement rack
- 130: anchor shaft
- 132: pusher rod locking tabs
- 230: anchor shaft
- 295: pendulum
- 296: anchor arm
- 297: anchor body
- 298: anchor mass
- 299: anchor

## Claims

1. An intraocular lens (IOL) injector (10) for releasing an IOL (32) to be injected into a capsular bag of an eye, the IOL injector (10) having a proximal end (30) and a distal end (24), the IOL injector comprising:
an injector body (12) defining a longitudinal axis and including a hollow shaft (102);
a spring arranged in the hollow shaft (102);
a cartridge (20) arranged on the injector body (12) and defining a cavity (18) for holding the IOL (32);
an injection nozzle (22) arranged on the cartridge (20) at the distal end (24) of the IOL injector;
a channel (14) extending through the injector body (12), the cavity (18), the cartridge (20), and the injection nozzle (22) in an axial direction along the longitudinal axis (P);
a mechanical speed regulator system (50) configured to control a speed of the releasing of the IOL;
a pusher rod (16) engaged via the hollow shaft (102) with the mechanical speed regulator system (50) and arranged in the channel (14) movably in the axial direction by a force generated by the spring (100) to advance the IOL (32) in the axial direction (P) towards the distal end of the IOL injector (10) out of the cavity (18) through the cartridge (20) and the injection nozzle (22);
a control lever (110) arranged on the injector body (12) in communication with the mechanical speed regulator system (50);
the mechanical speed regulator system (50) being responsive to an actuation of the control lever (110);
the mechanical speed regulator system (50) includes a first compound gear (52), a second compound gear (60), and a third compound gear (70, 90),
the first compound gear (52) includes a first pinion (55) and the hollow shaft (102) includes a rack (54);
the first pinion (55) and the rack (54) form a rack and pinion mechanism (51) to mechanically couple the mechanical speed regulator system (50) with the hollow shaft (102),
the first, second, and third compound gears form a gear train (92), and
the gear train (92) has a speed multiplying configuration;
**characterised in that**
the mechanical speed regulator system (50) further includes an anchor mechanism with an anchor (99), and
the third compound gear (90) includes an escapement wheel (94) mechanically engaging with the anchor (99).

2. The IOL injector of claim 1, wherein:
the anchor mechanism includes an anchor shaft (130) defining a center of oscillation of the anchor (99),
the anchor (99) has an anchor body (97), and
the anchor body is mounted on the anchor shaft (130) and swivels about the anchor shaft (130).

3. The IOL injector of claim 2, wherein:
the anchor mechanism further includes a pendulum arm (96) arranged on the anchor body (97) and a mass (98) arranged on an end of the pendulum arm (96) facing away from the anchor body (97).

## Patentansprüche

1. Intraokularlinsen (IOL)-Injektor (10) zur Freisetzung einer in einen Kapselsack eines Auges zu injizierenden IOL (32), wobei der IOL-Injektor (10) ein proximales Ende (30) und ein distales Ende (24) hat, wobei der IOL-Injektor umfasst:
einen Injektorkörper (12), der eine Längsachse definiert und einen hohlen Schaft (102) aufweist;
eine Feder, die in dem hohlen Schaft (102) angeordnet ist;
eine Kartusche (20), die an dem Injektorkörper (12) angeordnet ist und einen Hohlraum (18) zum Halten der IOL (32) definiert;
eine Einspritzdüse (22), die an der Kartusche (20) an dem distalen Ende (24) des IOL-Injektors angeordnet ist,
einen Kanal (14), der sich durch den Injektorkörper (12), den Hohlraum (18), die Kartusche (20) und die Einspritzdüse (22) in eine axiale Richtung entlang der Längsachse (P) erstreckt;
ein mechanisches Geschwindigkeitsregulierungssystem (50), das dazu ausgelegt ist, eine Geschwindigkeit der Freisetzung der IOL zu steuern;
eine Schubstange (16), die über den hohlen Schaft (102) mit dem mechanischen Geschwindigkeitsregulierungssystem (50) im Eingriff ist und in dem Kanal (14) in die axiale Richtung beweglich angeordnet ist durch eine Kraft, die von der Feder (100) erzeugt wird, um die IOL (32) in die axiale Richtung (P) in Richtung des distalen Endes des IOL-Injektors (10) aus dem Hohlraum (18) heraus durch die Kartusche (20) und in die Einspritzdüse (22) vorzuschieben;
einen Steuerhebel (110), der an dem Injektorkörper (12) in Verbindung mit dem mechanischen Geschwindigkeitsregulierungssystem (50) angeordnet ist;
wobei das mechanische Geschwindigkeitsregulierungssystem (50) auf die Betätigung des Steuerhebels (110) reagiert;
wobei das mechanische Geschwindigkeitsregulierungssystem (50) ein erstes Verbundzahnrad (52), ein zweites Verbundzahnrad (60) und ein drittes Verbundzahnrad (70, 90) aufweist,
wobei das erste Verbundzahnrad (52) ein erstes Ritzel (55) aufweist und der hohle Schaft (102) eine Zahnstange (54) aufweist;
wobei das erste Ritzel (55) und die Zahnstange (54) einen Zahnstangenmechanismus (51) bilden, um das mechanische Geschwindigkeitsregulierungssystem (50) mechanisch mit dem hohlen Schaft (102) zu koppeln,
wobei das erste, das zweite und das dritte Verbundzahnrad einen Getriebezug (92) bilden und der Getriebezug (92) eine geschwindigkeitsmultiplizierende Auslegung hat;
**dadurch gekennzeichnet, dass**
das mechanische Geschwindigkeitsregulierungssystem (50) ferner einen Verankerungsmechanismus mit einem Anker (99) aufweist, und
das dritte Verbundzahnrad (90) ein Ankerrad (94) aufweist, das mechanisch den Anker (99) in Eingriff nimmt.

2. IOL-Injektor nach Anspruch 1, wobei:
der Verankerungsmechanismus einen Ankerschaft (130) aufweist, der ein Zentrum der Schwingung des Ankers (99) definiert,
der Anker (99) einen Ankerkörper (97) hat, und
der Ankerkörper an dem Ankerschaft (130) montiert ist und um den Ankerschaft (130) schwenkt.

3. IOL-Injektor nach Anspruch 2, wobei:
der Ankermechanismus ferner einen Pendelarm (96) aufweist, der an dem Ankerkörper (97) angeordnet ist, und ein Gewicht (98), das an dem Pendelarm (96) angeordnet und von dem Ankerkörper (97) abgewandt ist.

## Revendications

1. Injecteur (10) de lentille intraoculaire (LIO) permettant de libérer une LIO (32) devant être injectée dans un sac capsulaire d'un œil, l'injecteur (10) de LIO présentant une extrémité proximale (30) et une extrémité distale (24), l'injecteur de LIO comprenant :
un corps d'injecteur (12) définissant un axe longitudinal et comprenant un arbre creux (102) ;
un ressort agencé dans l'arbre creux (102) ;
une cartouche (20) agencée sur le corps d'injecteur (12) et définissant une cavité (18) destinée à contenir la LIO (32) ;
une buse d'injection (22) agencée sur la cartouche (20) au niveau de l'extrémité distale (24) de l'injecteur de LIO ;
un canal (14) s'étendant à travers le corps d'injecteur (12), la cavité (18), la cartouche (20) et la buse d'injection (22) dans une direction axiale le long de l'axe longitudinal (P) ;
un système de régulateur de vitesse mécanique (50) conçu pour commander une vitesse de libération de la LIO ;
une tige de poussée (16) en prise par le biais de l'arbre creux (102) avec le système de régulateur de vitesse mécanique (50) et agencée dans le canal (14) de manière mobile dans la direction axiale par une force générée par le ressort (100) pour faire avancer la LIO (32) dans la direction axiale (P) vers l'extrémité distale de l'injecteur (10) de LIO hors de la cavité (18) à travers la cartouche (20) et la buse d'injection (22) ;
un levier de commande (110) agencé sur le corps d'injecteur (12) en communication avec le système de régulateur de vitesse mécanique (50) ;
le système de régulateur de vitesse mécanique (50) réagissant à un actionnement du levier de commande (110) ;
le système de régulateur de vitesse mécanique (50) comprend un premier engrenage composé (52), un deuxième engrenage composé (60) et un troisième engrenage composé (70, 90),
le premier engrenage composé (52) comprend un premier pignon (55) et l'arbre creux (102) comprend une crémaillère (54) ;
le premier pignon (55) et la crémaillère (54) forment un mécanisme à pignon et crémaillère (51) pour accoupler mécaniquement le système de régulateur de vitesse mécanique (50) avec l'arbre creux (102),
les premier, deuxième et troisième engrenages composés forment un train d'engrenages (92), et
le train d'engrenages (92) présente une configuration de multiplication de vitesse ;
caractérisé en ce fait que
le système de régulateur de vitesse mécanique (50) comprend en outre un mécanisme d'ancrage doté d'un ancrage (99), et
le troisième engrenage composé (90) comprend une roue d'échappement (94) entrant en prise mécaniquement avec l'ancrage (99).

2. Injecteur de LIO selon la revendication 1, dans lequel :
le mécanisme d'ancrage comprend un arbre d'ancrage (130) définissant un centre d'oscillation de l'ancrage (99),
l'ancrage (99) présente un corps d'ancrage (97), et
le corps d'ancrage est monté sur l'arbre d'ancrage (130) et pivote autour de l'arbre d'ancrage (130).

3. Injecteur de LIO selon la revendication 2, dans lequel :
le mécanisme d'ancrage comprend en outre un bras pendulaire (96) agencé sur le corps d'ancrage (97) et une masse (98) agencée sur une extrémité du bras pendulaire (96) orientée à l'opposé du corps d'ancrage (97).
